# EUROPEAN PATENT SPECIFICATION

(11) **EP 3 352 854 B1**
(45) Date of publication and mention of the grant of the patent: **15.05.2019**
(21) Application number: 17742754.9
(22) Date of filing: 24.07.2017
(51) Int. Cl.: A61N 7/02, G01K 11/32, G01K 13/00, A61B 17/00, A61B 18/00, A61B 5/00, A61B 5/01

(54) **BLADDER TEMPERATURE MEASUREMENT FOR HIGH INTENSITY FOCUSED ULTRASOUND**
BLASENTEMPERATURMESSUNG FÜR HOCHINTENSIV FOKUSSIERTEN ULTRASCHALL
MESURE DE LA TEMPÉRATURE DE LA VESSIE POUR ULTRASONS FOCALISÉS DE HAUTE INTENSITÉ

(30) Priority: 25.07.2016 EP 16180945
(43) Date of publication of application: 01.08.2018
(73) Proprietor: Koninklijke Philips N.V., 5656 AE Eindhoven (NL)
(72) Inventor: HEIJMAN, Edwin, 5656 AE Eindhoven (NL); VAN DER MARK, Martinus, Bernardus, 5656 AE Eindhoven (NL)
(74) Representative: van Iersel, Hannie
(86) International application number: PCT/EP2017/068669
(87) International publication number: WO 2018/019781

(56) References cited:
- EP-A1- 2 627 412
- EP-A1- 2 836 274
- WO-A1-2013/153509
- WO-A2-2007/106680
- US-A- 4 215 275
- US-A- 4 785 824
- US-A1- 2012 203 098
- US-A1- 2015 157 713

## Description

### FIELD OF THE INVENTION

The invention relates to High Intensity Focused Ultrasound, in particular to the measurement of body temperature prior to and/or during sonication.

### BACKGROUND OF THE INVENTION

Ultrasound from a focused ultrasonic transducer can be used to selectively treat regions within the interior of the body. Ultrasonic waves are transmitted as high energy mechanical vibrations. These vibrations induce tissue heating as they are damped, and they can also lead to cavitation. Both tissue heating and cavitation can be used to destroy tissue in a clinical setting. However, heating tissue with ultrasound is easier to control than cavitation. Ultrasonic treatments can be used to ablate tissue and to kill regions of cancer cells selectively. This technique has been applied to the treatment of uterine fibroids, and has reduced the need for hysterectomy procedures.

To selectively treat tissue, a focused ultrasonic transducer can be used to focus the ultrasound on a particular treatment or target volume. The transducer is typically mounted within a medium, such as oil or degassed water, that is able to transmit ultrasound. Actuators are then used to adjust the position of the ultrasonic transducer and thereby adjust the tissue region that is being treated. The guiding of ultrasound therapy using magnetic resonance imaging is known.

United States Patent Publication US 8,233,957 B2 discloses a sensor module for a catheter, the sensor module comprising a biofilm detection unit adapted for detecting a characteristic of a biofilm and electrical circuitry for providing an output signal indicative of a result of the detection.

Documents WO2007106680 A2, US4785824 A, US4215275 A, WO2013153509 A1, US2015157713 A1, EP2836274 A2, EP2627412 A2 and US2012203098 A1 disclose further relevant background art.

### SUMMARY OF THE INVENTION

The invention provides for a medical instrument and a computer program product according to the independent claims. Embodiments are given in the dependent claims.

Before a High Intensity Focused Ultrasound (HIFU) sonication the body temperature may be measured as part of the planning process for performing a sonication. For example, the body temperature is used as a baseline for presenting absolute temperatures to the user during the sonication. During a HIFU sonication portions of a subject have their temperature raised within a target zone to a set temperature for a particular duration. Knowing what the body temperature is may be useful in this planning.

In a clinical setting the measurement of the body temperature is typically performed by measuring the ear temperature. This may lead to several difficulties. First if the sonication is performed on an abdominal region or other region away from the ear this may result in an inaccurate estimate of the body's core temperature. Another difficulty is that during the course of a sonication the body temperature typically changes, and it is not necessarily due to the sonication of the patient. The patient may partially disrobe for the sonication which over time may lead to a change in body temperature. Also, a frequent complaint during a sonication is that the subject becomes cold. A blanket may sometimes be provided to the subject which can also introduce a drift in the body temperature.

Examples not according to the inventions may possibly provide for an improved method of measuring body temperature that is HIFU compatible. A temperature sensitive fluorescent dye can be injected into the bladder of the subject. A temperature sensitive fluorescent dye is a dye that measurably different light frequencies, excited-state lifetime and/or fluorescence emission in responses to changes in temperature. A urinary catheter with an optical fiber cable can be used to excite the temperature sensitive fluorescent dye within the bladder. A light sensor or spectrometer that is coupled to the optical fiber cable can then be used to make a spectrograph or light intensity measured at specific wavelength(s) of the fluoresced light, which can be used to calculate the temperature within the bladder. This may provide an accurate means of measuring the temperature of fluid within the bladder. This bladder temperature is an extremely accurate measurement of the body's core temperature.

This technique can also be useful for sonications that are close to the bladder or when ultrasonic waves travel through the bladder. For example if a prostrate or uterine fibroids are being sonicated the measured temperature of the bladder will be nearly identical with the temperature of the intended sonication target.

Measuring the fluoresced light within the bladder may provide several advantages. Firstly the fluid within the bladder will quickly come to thermal equilibrium. If a portion of the bladder is exposed to (scattered) ultrasound, the scattered ultrasound will only have a minor effect on the urine temperature. Firstly because of the aforementioned tendency to come to thermal equilibrium. Secondly because the catheter gathers light from a portion of the bladder. There is a spatial averaging effect when the temperature is measured. Thirdly, the ultrasonic waves will only heat the urine by a small amount due to it's low absorption of ultrasonic energy.

The effectiveness of the temperature measurement can be increased by adding a scattering medium made of particulates into the fluid in the bladder also. This may help to spread light during the illumination of the temperature sensitive fluorescent dye and increase the overall signal. The scattering material may also serve to increase the coupling of fluorescing light emitted by the temperatures sensitive fluorescent dye into the optical fiber cable. The scattering medium may therefore have the benefit of increasing the spatial averaging of the temperature measurement of fluid within the bladder.

In one aspect the invention provides for a medical instrument comprising a high-intensity focused ultrasound system for sonicating a target region within a subject. The medical instrument further comprises a light source for exciting a temperature-sensitive fluorescent dye. The light source is configured for coupling to an optical fiber cable of a urinary catheter. The medical instrument further comprises a light sensor, which may be in one example a spectrometer, for measuring a fluorescence emitted by the temperature-sensitive fluorescent dye. If the light sensor is a spectrometer, then the fluorescence may be a florescence spectrum.

It is understood herein that references to a light sensor may also refer to various instrument such as a monochrometer or spectrometer and that references to "fluorescence" may refer to measured fluorescence light data such as an overall intensity of measured fluorescence or light within one or more wavelength ranges. References to "fluorescence" may also refer to fluorescence spectrums measured by a spectrometer or a band of light measured by a monochrometer.

The light sensor or spectrometer is configured for coupling to the optical fiber cable. The medical instrument further comprises a memory for storing machine-executable instructions. The medical instrument further comprises a processor for controlling the medical instrument. Execution of the machine-executable instructions causes the processor to receive a sonication plan descriptive of the sonication of the target region.

Execution of the machine-executable instructions further causes the processor to measure the fluorescence using the light sensor. In some examples the light source may be always active. In this case it would not be necessary for the processor to control the light source. In other examples the light source is activated shortly before the fluorescence is measured. In this case it may be that the processor controls the light source. However, this is not necessary. Execution of the machine-executable instructions further causes the processor to calculate a bladder temperature using the fluorescence spectrum or intensity. The fluorescence of the temperature-sensitive fluorescent dye may have a frequency shift or different emission intensity depending upon the temperature. By measuring the fluorescence with a light sensor such as a spectrometer or the intensity this frequency shift and/or emission change can be measured and thereby a bladder temperature can be calculated or deduced. Execution of the machine-executable instructions further cause the processor to generate sonication commands using the sonication plan and the bladder temperature. The sonication commands are adapted for controlling the high-intensity focused ultrasound system to sonicate the target region.

When sonications are performed using high-intensity focused ultrasound systems a body core temperature is typically provided to generate sonication commands. This for example may be measured using a measurement of an ear temperature. The same techniques may be used to generate the sonication commands in this example. However, using the bladder temperature may be more accurate. The bladder is within the core of the body. This may provide for a more accurate measurement of the body's core temperature. For sonications near the vicinity of the bladder using the bladder temperature may be even better because it may be more representative of the region of the subject which is to be sonicated.

Measuring the bladder temperature may be further beneficial because if it is filled with a fluid such as urine or material which has been added to the bladder, it may have a reasonable thermal inertia. For example if a region in the vicinity of the bladder is being sonicated a portion of the bladder may be heated by the sonication. This however will not effect the overall temperature of the fluid within the bladder because of the high thermal conductivity of water. It may therefore be beneficial to measure the bladder temperature as opposed to some other body region when sonicating near the bladder to determine the core or base temperature of the body of the subject. Another advantage is that the absorption of ultrasonic energy by urine is is comparable with water, and is low in comparison with body tissues.

In various examples, the optical fiber cable may comprise one or more optical fibers. For example a single optical fiber may be used and both the light source and the spectrometer or light sensor may be coupled to it. If the emission of the fluorescence is far enough away from the frequency of light source then a single fiber optic can be used. In other examples there may be two separate fiber optics. For example there may be one fiber which is connected to the light source and one which is connected to the light sensor or spectrometer.

In some examples; the light source is on continuously. In other examples the machine-executable instructions may cause the processor to control the light source to eliminate the distal end of the urinary catheter before the fluorescence is measured.

In another embodiment, the target zone is within a predetermined distance within the subject from the bladder.

In another embodiment, the distal end of the catheter may have one or more optical elements which are coupled to the optical fiber cable. For instance, there may be lenses which enable the light to be spread to a larger region of the bladder. Also there may be a lens which enables the capturing of more light for by the light sensor or spectrometer. There may also be polarizing filters to help to exclude particular portions of the light. If there are two fibers within the optical fiber cable the optical fiber connected to the light sensor may have a filter to filter out light from the light source.

In another embodiment, the medical instrument comprises the urinary catheter. The optical fiber extends from a distal end of the urinary catheter along a length of the urinary catheter. The optical fiber cable may for instance be embedded within the urinary catheter. The optical fiber cable is coupled to the light source. The optical fiber cable is further coupled to the spectrometer or light sensor. The light source is configured to eliminate the distal end of the urinary catheter using the optical fiber cable.

In another embodiment, the medical imaging system further comprises a fluorescent dye reservoir. The medical imaging system further comprises a pump system connected to the urinary catheter. The pump system is further connected to the fluorescent mixture. The pump system is configured for pumping from the fluorescent dye reservoir to the distal end of the urinary catheter. This embodiment may be beneficial because it may provide for a means of efficiently adding the fluorescent dye and/or scattering agent to the bladder of the subject. The pump system for example could be either manually controlled or automatically controlled.

The pump system could function differently in different configurations. In one example the catheter has a main tube. In one example, the catheter only has the main tube. The main tube may be used to remove urine from the bladder of the subject and it also may be used to pump the contents of a fluorescent dye reservoir back into the bladder of the subject. The pump could alternately remove material and pump it into a waste reservoir and fill material back into the bladder from the fluorescent dye reservoir.

In another example, the urinary catheter has a main tube for removing urine and other contents of the bladder to the waste reservoir and an auxiliary tube for pumping material from the fluorescent dye reservoir into the bladder. Having a main tube and an auxiliary tube in the urinary catheter may be beneficial because this may also provide for a means of continually mixing the temperature-sensitive fluorescent dye and scattering medium within the bladder. For example the pump system can be configured to remove material using the main tube and pump material in using the auxiliary tube at a low but constant rate so that the contents of the bladder are continually mixed. This may also allow the control of the concentration of any scattering agent and the temperature-sensitive fluorescent dye within the bladder of the subject.

In another embodiment, execution of the machine-executable instructions further cause the processor to control the pump system to pump contents of the fluorescent dye reservoir to the distal end of the urinary catheter if the fluorescence or the fluorescence spectrum has a magnitude below a predetermined threshold. The temperature within the bladder may be measured by measuring a temperature shift or intensity change of the temperature-sensitive fluorescent dye. However, if the signal is too low the measurement may be poor. The processor can be programmed to look at the magnitude or strength of the signal coming from the spectrometer or light sensor. If the fluorescence or the fluorescence spectrum has a magnitude below the predetermined threshold then more temperature-sensitive fluorescent dye can be automatically added to the bladder of the subject. Adding fluid may also be used to increase the volume of the bladder.

In another embodiment, the fluorescent dye reservoir contains the temperature-sensitive fluorescent dye.

In another embodiment, the fluorescent dye reservoir contains scattering material.

In another embodiment, the fluorescent dye reservoir contains the temperature-sensitive fluorescent dye and the scattering material.

In another embodiment, the scattering material comprises any one of the following: titanium dioxide particles, an ultrasound contrast medium, lipid droplets, milk droplets, soya droplets, intralipid droplets, latex particles, and combinations thereof. The scattering material may be practically any material which may be used for scattering the light generated by the light source and/or the fluorescence spectra emitted by the temperature-sensitive fluorescent dye. Placing the scattering material in the bladder of the subject may be useful for better coupling the light source and the spectrometer or light sensor to the bladder. It may enable the gathering of a larger amount of emitted fluorescence or the fluorescence spectrum.

The ultrasound contrast medium for example may be used in a concentration of approximately 0.1g per liter (roughly 0.03g to 0.5g per liter).

The titanium dioxide particles may have a diameter of approximately 250 nm (approximately 100 to 500 nm) in some examples. They may also be used in a density of approximately 0.1g per liter (approximately 0.03 g per liter to 0.5 g per liter) of fluid in the bladder.

The latex particles in one example may have a diameter of approximately 10 µm (approximately 3 µm to 50 µm). The latex particles may for instance be used in a concentration of approximately 1g per liter (approximately 0.3g to 5g per liter).

In one example lipide droplets (mil, soya, Intralipid) having approximately a diameter from 50 nm to 10 µm or a mixture of different diameters. These droplets can be used in a concentration of 1-20 volume%.

Glycerin can be used in a concentration of approximately 10 volume% (can range from 1-50 volume%) in one example.

In another embodiment the temperature sensitive fluorescent dye is any one of the following: Fluorescein, Fluorescein 1, Fluorescein Sodium, Indocyanine green, Photofrin, , 5-aminolevulinic acid (ALA) and methylene blue.

Fluorescein sodium has an excitation of about 480 nm. The emission spectrum has a center at about 525 nm which varies slightly for temperature. Various concentrations of fluorescein sodium may be used. When used for angiography typically 500 mg are injected into the adult which has 6 liters of blood. This results in a concentration of 83 mg per liter. This is a fairly high concentration since 80% of the dye will typically bind to plasma proteins in the blood. When used in the bladder the fluorescein may be used at a considerably lower concentration. For example a concentration of about 5-20 mg per liter of fluid (alternatively 1 to 50 mg per liter of fluid) within the bladder may work well. Fluorescein is used as fluorescent tracer and medical applied in ophthalmology and has a temperature depended frequency shift

Indocyanine green (ICG) is used as tracer in angiography and ophthalmology and binds strongly to plasma proteins and exhibits aqueous, photo, and is thermal instable.

Photofrin is used for photodynamic therapy. It has a temperature depended quantum yield. It has a slide frequency shift at high pH values at function of temperature.

5-aminolevulinic acid (ALA) is used for photodynamic therapy and has increased fluorescence as function of temperature. If ALA is used then the bladder may possibly be continuously filled and emptied with a solution that contains a known concentration of ALA so that the intensity of the fluorescent spectrum is calibrated.

In another embodiment, the sonication commands are further calculated by using the bladder temperature as a starting temperature for a sonication. As mentioned above, when performing sonications typically the doctor or medical technician will measure the body core temperature through the ear. This is then used in the calculations for calculating the sonication commands. The temperature in the ear may be inaccurate and also during the examination the core body temperature of the subject may increase. Typically when the sonications are performed the patient or subject may become uncomfortable. It is not uncommon for the medical technician to place a blanket over the subject. During the course of the sonication this may cause a change in the core temperature or starting temperature of the subject for individual sonications. Measuring the bladder temperature throughout the course of the sonications may provide for more accurate sonication of the subject.

In another embodiment, the sonication plan comprises a specified thermal dose. The sonication commands are calculated to deliver the specified thermal dose to the target region using the starting temperature. Using the bladder temperature as a starting temperature or body core temperature may enable more accurate calculation of the thermal dose or prediction of the thermal dose when calculating the sonication commands.

In another embodiment, execution of the machine-executable instructions further causes the processor to control the high-intensity focused ultrasound system using the sonication commands.

In another embodiment, execution of the machine-executable instructions further causes the processor to repeatedly calculate the bladder temperature during controlling of the high-intensity focused ultrasound system. Execution of the machine-executable instructions further cause the processor to repeatedly correct the sonication commands using the bladder temperature during controlling of the high-intensity focused ultrasound system. This embodiment may be beneficial because the measurement of the bladder temperature forms a closed control loop during the sonication of the subject.

In another embodiment, the sonication commands specify multiple sonications. The measurement of the bladder temperature and the generation of the sonication commands are repeated at least once for each of the multiple sonications. Repeatedly measuring the bladder temperature and using this as a core body temperature or initial temperature may enable more accurate sonications to be performed.

In another embodiment, the high-intensity focused ultrasound system has an adjustable focus. The medical instrument further comprises a medical imaging system. Execution of the machine-executable instructions further causes the processor to acquire a medical image from an imaging zone. The imaging zone comprises the target region. The sonication commands are adapted for controlling the location of the adjustable focus. It may be beneficial to use a medical imaging system for guiding the sonication. In various examples the medical imaging system could for example be a magnetic resonance imaging system, a diagnostic ultrasound system, or even a radiographic imaging system such as a CT or computer tomography system. The use of a magnetic resonance imaging system may be beneficial because the magnetic resonance imaging system could for example perform magnetic resonance thermometry and be used to measure the thermal dose delivered to the subject. Also the bladder temperature could also be used to calibrate a temperature measurement using the magnetic resonance thermometry.

In another aspect, the invention provides for a computer program product comprising machine-executable instructions for execution by a processor controlling the medical instrument. The medical instrument comprises a high-intensity focused ultrasound system for sonicating a target region within a subject. The medical instrument further comprises a light source for exciting a temperature-sensitive fluorescent dye. The light source is configured for coupling to an optical fiber cable of a urinary catheter. The medical instrument further comprises a light sensor or spectrometer for measuring a fluorescence or a fluorescence spectrum emitted by the temperature-sensitive fluorescent dye. The light sensor or spectrometer is configured for coupling to the optical fiber cable.

Execution of the machine-executable instructions causes the processor to receive a sonication plan descriptive of the sonication of the target region. Execution of the machine-executable instructions further causes the processor to measure the fluorescence or the fluorescence spectrum using the light sensor or spectrometer. Execution of the machine-executable instructions further causes the processor to calculate a bladder temperature using the fluorescence. Execution of the machine-executable instructions further cause the processor to generate sonication commands using the sonication plan and the bladder temperature. The sonication commands are adapted for controlling the high-intensity focused ultrasound system to sonicate the target region.

In another embodiment the medical instrument further comprises the urinary catheter.

In another embodiment not according to the invention, the disclosure provides for a method of operating a medical instrument. The medical instrument comprises a high-intensity focused ultrasound system for sonicating a target region within a subject. The medical instrument further comprises a light source for exciting a temperature-sensitive fluorescent dye. The light source is configured for coupling to an optical fiber cable of a urinary catheter. The medical instrument further comprises a light sensor or spectrometer for measuring fluorescence or a fluorescence spectrum emitted by the temperature-sensitive fluorescent dye. The light sensor spectrometer is configured for coupling to the optical fiber cable.

The method comprises receiving a sonication plan descriptive of a sonication of the target region. The method further comprises measuring the fluorescence or fluorescence spectrum using the light sensor or spectrometer. The method further comprises calculating a bladder temperature using the fluorescence. The method further comprises generating sonication commands using the sonication plan and the bladder temperature. The sonication commands are adapted for controlling the high-intensity focused ultrasound system to sonicate the target region.

In another embodiment, the medical instrument further comprises the urinary catheter.

In some examples, the bladder could already be filled with fluorescent dye and optionally scattering material. This may be done manually. In other embodiments a pump system may be used to pump the fluorescent dye and optionally the scattering material into the bladder of the subject.

It is understood that one or more of the aforementioned embodiments of the invention may be combined as long as the combined embodiments are not mutually exclusive.

As will be appreciated by one skilled in the art, aspects of the present invention may be embodied as an apparatus or computer program product. Accordingly, aspects of the present invention may take the form of an entirely hardware embodiment, an entirely software embodiment (including firmware, resident software, microcode, etc.) or an embodiment combining software and hardware aspects that may all generally be referred to herein as a "circuit," "module" or "system." Furthermore, aspects of the present invention may take the form of a computer program product embodied in one or more computer readable medium(s) having computer executable code embodied thereon.

Any combination of one or more computer readable medium(s) may be utilized. The computer readable medium may be a computer readable signal medium or a computer readable storage medium. A 'computer-readable storage medium' as used herein encompasses any tangible storage medium which may store instructions which are executable by a processor of a computing device. The computer-readable storage medium may be referred to as a computer-readable non-transitory storage medium. The computer-readable storage medium may also be referred to as a tangible computer readable medium. In some embodiments, a computer-readable storage medium may also be able to store data which is able to be accessed by the processor of the computing device. Examples of computer-readable storage media include, but are not limited to: a floppy disk, a magnetic hard disk drive, a solid state hard disk, flash memory, a USB thumb drive, Random Access Memory (RAM), Read Only Memory (ROM), an optical disk, a magneto-optical disk, and the register file of the processor. Examples of optical disks include Compact Disks (CD) and Digital Versatile Disks (DVD), for example CD-ROM, CD-RW, CD-R, DVD-ROM, DVD-RW, or DVD-R disks. The term computer readable-storage medium also refers to various types of recording media capable of being accessed by the computer device via a network or communication link. For example a data may be retrieved over a modem, over the internet, or over a local area network. Computer executable code embodied on a computer readable medium may be transmitted using any appropriate medium, including but not limited to wireless, wire line, optical fiber cable, RF, etc., or any suitable combination of the foregoing.

A computer readable signal medium may include a propagated data signal with computer executable code embodied therein, for example, in baseband or as part of a carrier wave. Such a propagated signal may take any of a variety of forms, including, but not limited to, electro-magnetic, optical, or any suitable combination thereof. A computer readable signal medium may be any computer readable medium that is not a computer readable storage medium and that can communicate, propagate, or transport a program for use by or in connection with an instruction execution system, apparatus, or device.

'Computer memory' or 'memory' is an example of a computer-readable storage medium. Computer memory is any memory which is directly accessible to a processor. 'Computer storage' or 'storage' is a further example of a computer-readable storage medium. Computer storage may be any volatile or non-volatile computer-readable storage medium.

A 'processor' as used herein encompasses an electronic component which is able to execute a program or machine executable instruction or computer executable code. References to the computing device comprising "a processor" should be interpreted as possibly containing more than one processor or processing core. The processor may for instance be a multi-core processor. A processor may also refer to a collection of processors within a single computer system or distributed amongst multiple computer systems. The term computing device should also be interpreted to possibly refer to a collection or network of computing devices each comprising a processor or processors. The computer executable code may be executed by multiple processors that may be within the same computing device or which may even be distributed across multiple computing devices.

Computer executable code may comprise machine executable instructions or a program which causes a processor to perform an aspect of the present invention. Computer executable code for carrying out operations for aspects of the present invention may be written in any combination of one or more programming languages, including an object oriented programming language such as Java, Smalltalk, C++ or the like and conventional procedural programming languages, such as the C programming language or similar programming languages and compiled into machine executable instructions. In some instances the computer executable code may be in the form of a high level language or in a pre-compiled form and be used in conjunction with an interpreter which generates the machine executable instructions on the fly.

The computer executable code may execute entirely on the user's computer, partly on the user's computer, as a stand-alone software package, partly on the user's computer and partly on a remote computer or entirely on the remote computer or server. In the latter scenario, the remote computer may be connected to the user's computer through any type of network, including a local area network (LAN) or a wide area network (WAN), or the connection may be made to an external computer (for example, through the Internet using an Internet Service Provider).

Aspects of the present invention are described with reference to flowchart illustrations and/or block diagrams of apparatus (systems) and computer program products according to embodiments of the invention. It is understood that each block or a portion of the blocks of the flowchart, illustrations, and/or block diagrams, can be implemented by computer program instructions in form of computer executable code when applicable. It is further understood that, when not mutually exclusive, combinations of blocks in different flowcharts, illustrations, and/or block diagrams may be combined. These computer program instructions may be provided to a processor of a general purpose computer, special purpose computer, or other programmable data processing apparatus to produce a machine, such that the instructions, which execute via the processor of the computer or other programmable data processing apparatus, create means for implementing the functions/acts specified in the flowchart and/or block diagram block or blocks.

These computer program instructions may also be stored in a computer readable medium that can direct a computer, other programmable data processing apparatus, or other devices to function in a particular manner, such that the instructions stored in the computer readable medium produce an article of manufacture including instructions which implement the function/act specified in the flowchart and/or block diagram block or blocks.

The computer program instructions may also be loaded onto a computer, other programmable data processing apparatus, or other devices to cause a series of operational steps to be performed on the computer, other programmable apparatus or other devices to produce a computer implemented process such that the instructions which execute on the computer or other programmable apparatus provide processes for implementing the functions/acts specified in the flowchart and/or block diagram block or blocks.

A 'user interface' as used herein is an interface which allows a user or operator to interact with a computer or computer system. A 'user interface' may also be referred to as a 'human interface device.' A user interface may provide information or data to the operator and/or receive information or data from the operator. A user interface may enable input from an operator to be received by the computer and may provide output to the user from the computer. In other words, the user interface may allow an operator to control or manipulate a computer and the interface may allow the computer indicate the effects of the operator's control or manipulation. The display of data or information on a display or a graphical user interface is an example of providing information to an operator. The receiving of data through a keyboard, mouse, trackball, touchpad, pointing stick, graphics tablet, joystick, gamepad, webcam, headset, pedals, wired glove, remote control, and accelerometer are all examples of user interface components which enable the receiving of information or data from an operator.

A 'hardware interface' as used herein encompasses an interface which enables the processor of a computer system to interact with and/or control an external computing device and/or apparatus. A hardware interface may allow a processor to send control signals or instructions to an external computing device and/or apparatus. A hardware interface may also enable a processor to exchange data with an external computing device and/or apparatus. Examples of a hardware interface include, but are not limited to: a universal serial bus, IEEE 1394 port, parallel port, IEEE 1284 port, serial port, RS-232 port, IEEE-488 port, bluetooth connection, wireless local area network connection, TCP/IP connection, ethernet connection, control voltage interface, MIDI interface, analog input interface, and digital input interface.

A 'display' or 'display device' as used herein encompasses an output device or a user interface adapted for displaying images or data. A display may output visual, audio, and or tactile data. Examples of a display include, but are not limited to: a computer monitor, a television screen, a touch screen, tactile electronic display, Braille screen, Cathode ray tube (CRT), Storage tube, Bi-stable display, Electronic paper, Vector display, Flat panel display, Vacuum fluorescent display (VF), Light-emitting diode (LED) display, Electroluminescent display (ELD), Plasma display panel (PDP), Liquid crystal display (LCD), Organic light-emitting diode display (OLED), a projector, and Head-mounted display.

Medical imaging data is defined herein as two or three dimensional data that has been acquired using a medical imaging system. A medical imaging system is defined herein as a apparatus adapted for acquiring information about the physical structure of a patient and construct sets of two dimensional or three dimensional medical imaging data. Medical imaging data can be used to construct visualizations which might be useful for diagnosis by a physician. This visualization can be performed using a computer.

Magnetic Resonance (MR) data is defined herein as being the recorded measurements of radio frequency signals emitted by atomic spins using the antenna of a magnetic resonance apparatus during a magnetic resonance imaging scan. Magnetic resonance data is an example of medical imaging data. A Magnetic Resonance (MR) image is defined herein as being the reconstructed two or three dimensional visualization of anatomic data contained within the magnetic resonance imaging data.

MR thermometry data may be understood as being the recorded measurements of radio frequency signals emitted by atomic spins by the antenna of a Magnetic resonance apparatus during a magnetic resonance imaging scan which contains information which may be used for magnetic resonance thermometry. Magnetic resonance thermometry functions by measuring changes in temperature sensitive parameters. Examples of parameters that may be measured during magnetic resonance thermometry are: the proton resonance frequency shift, the diffusion coefficient, or changes in the T1 and/or T2 relaxation time may be used to measure the temperature using magnetic resonance. The proton resonance frequency shift is temperature dependent, because the magnetic field that individual protons, hydrogen atoms, experience depends upon the surrounding molecular structure. An increase in temperature decreases molecular screening due to the temperature affecting the hydrogen bonds. This leads to a temperature dependence of the proton resonant frequency.

The proton density depends linearly on the equilibrium magnetization. It is therefore possible to determine temperature changes using proton density weighted images.

The relaxation times T1, T2, and T2-star (sometimes written as T2*) are also temperature dependent. The reconstruction of T1, T2, and T2-star weighted images can therefore be used to construct thermal or temperature maps.

The temperature also affects the Brownian motion of molecules in an aqueous solution. Therefore pulse sequences which are able to measure diffusion coefficients such as a pulsed diffusion gradient spin echo may be used to measure temperature.

One of the most useful methods of measuring temperature using magnetic resonance is by measuring the proton resonance frequency (PRF) shift of water protons. The resonant frequency of the protons is temperature dependent. As the temperature changes in a voxel the frequency shift will cause the measured phase of the water protons to change. The temperature change between two phase images can therefore be determined. This method of determining temperature has the advantage that it is relatively fast in comparison to the other methods. The PRF method is discussed in greater detail than other methods herein. However, the methods and techniques discussed herein are also applicable to the other methods of performing thermometry with magnetic resonance imaging.

An 'ultrasound window' as used herein encompasses a window which is able to transmit ultrasonic waves or energy. Typically a thin film or membrane is used as an ultrasound window. The ultrasound window may for example be made of a thin membrane of BoPET (Biaxially-oriented polyethylene terephthalate).

### BRIEF DESCRIPTION OF THE DRAWINGS

In the following preferred embodiments of the invention will be described, by way of example only, and with reference to the drawings in which:
- Fig. 1 illustrates and example of a medical instrument;
- Fig. 2 shows a flow chart which illustrates a method of using the medical instrument of Fig. 1;
- Fig. 3 illustrates an example of a urinary catheter;
- Fig. 4 shows a cross sectional view of the urinary catheter of Fig. 3 and 5;
- Fig. 5 illustrates a further example of a urinary catheter;
- Fig. 6 illustrates a further example of a urinary catheter;
- Fig. 7 shows a cross sectional view of the urinary catheter of Fig. 6;
- Fig. 8 illustrates a further example of a urinary catheter; and
- Fig. 9 shows a cross sectional view of the urinary catheter of Fig. 8;

### DETAILED DESCRIPTION OF THE EMBODIMENTS

Like numbered elements in these figures are either equivalent elements or perform the same function. Elements which have been discussed previously will not necessarily be discussed in later figures if the function is equivalent.

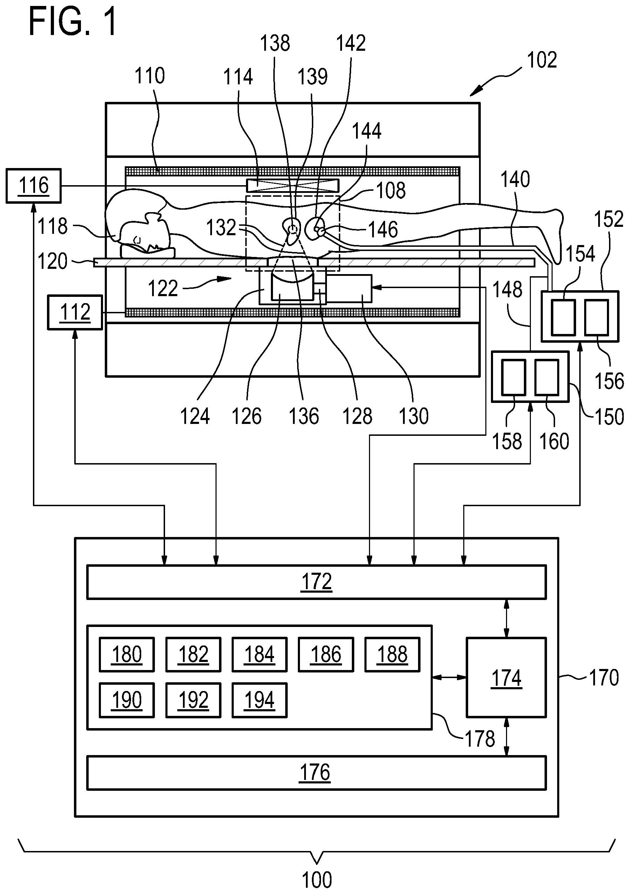

Fig. 1 shows an example of a medical instrument 100. The medical instrument 100 comprises a magnetic resonance imaging system 102. The magnetic resonance imaging system comprises a magnet 104. The magnet 104 is a cylindrical type superconducting magnet with a bore 106 through the center of it. The magnet has a liquid helium cooled cryostat with superconducting coils. It is also possible to use permanent or resistive magnets. The use of different types of magnets is also possible for instance it is also possible to use both a split cylindrical magnet and a so called open magnet. A split cylindrical magnet is similar to a standard cylindrical magnet, except that the cryostat has been split into two sections to allow access to the iso-plane of the magnet, such magnets may for instance be used in conjunction with charged particle beam therapy. An open magnet has two magnet sections, one above the other with a space in-between that is large enough to receive a subject: the arrangement of the two sections area similar to that of a Helmholtz coil. Open magnets are popular, because the subject is less confined. Inside the cryostat of the cylindrical magnet there is a collection of superconducting coils. Within the bore 106 of the cylindrical magnet there is an imaging zone 108 where the magnetic field is strong and uniform enough to perform magnetic resonance imaging.

Within the bore 106 of the magnet there is also a set of magnetic field gradient coils 110 which are used for acquisition of magnetic resonance data to spatially encode magnetic spins within the imaging zone 108 of the magnet 104. The magnetic field gradient coils are connected to a magnetic field gradient coil power supply 112. The magnetic field gradient coils 110 are intended to be representative. Typically magnetic field gradient coils contain three separate sets of coils for spatially encoding in three orthogonal spatial directions. A magnetic field gradient power supply 112 supplies current to the magnetic field gradient coils 110. The current supplied to the magnetic field coils is controlled as a function of time and may be ramped or pulsed.

Adjacent to the imaging zone 108 is a radio-frequency coil 114 for manipulating the orientations of magnetic spins within the imaging zone 108 and for receiving radio transmissions from spins also within the imaging zone. The radio-frequency coil may contain multiple coil elements. The radio-frequency coil may also be referred to as a channel or an antenna. The radio-frequency coil 114 is connected to a radio frequency transceiver 116. The radio-frequency coil 114 and radio frequency transceiver 116 may be replaced by separate transmit and receive coils and a separate transmitter and receiver. It is understood that the radio-frequency coil 114 and the radio-frequency transceiver 116 are representative. The radio-frequency coil 114 is intended to also represent a dedicated transmit antenna and a dedicated receive antenna. Likewise the transceiver 116 may also represent a separate transmitter and receivers.

A subject 118 is shown as reposing on a subject support 120 and is located partially within the imaging zone 108. The example shown in Fig. 1 comprises a high-intensity focused ultrasound system 122. The high-intensity focused ultrasound system comprises a fluid-filled chamber 124. Within the fluid-filled chamber 124 is an ultrasound transducer 126. Although it is not shown in this figure the ultrasound transducer 126 may comprise multiple ultrasound transducer elements each capable of generating an individual beam of ultrasound. This may be used to steer the location of a sonication point 138 electronically by controlling the phase and/or amplitude of alternating electrical current supplied to each of the ultrasound transducer elements. A target region 139 is also marked on Fig. 1. The sonication point 138 can be steered to sonicate the target region 139.

The ultrasound transducer 126 is connected to a mechanism 128 which allows the ultrasound transducer 126 to be repositioned mechanically. The mechanism 128 is connected to a mechanical actuator 130 which is adapted for actuating the mechanism 128. The mechanical actuator 130 also represents a power supply for supplying electrical power to the ultrasound transducer 126. In some embodiments the power supply may control the phase and/or amplitude of electrical power to individual ultrasound transducer elements. In some embodiments the mechanical actuator/power supply 130 is located outside of the bore 106 of the magnet 104.

The ultrasound transducer 126 generates ultrasound which is shown as following the path 132. The ultrasound 132 goes through the fluid-filled chamber 128 and through an ultrasound window 134. In this embodiment the ultrasound then passes through a gel pad 136. The gel pad 136 is not necessarily present in all embodiments but in this embodiment there is a recess in the subject support 120 for receiving a gel pad 136. The gel pad 136 helps couple ultrasonic power between the transducer 126 and the subject 118. After passing through the gel pad 136 the ultrasound 132 passes through the subject 118 and is focused to a sonication point 138. The sonication point 138 may be moved through a combination of mechanically positioning the ultrasonic transducer 126 and electronically steering the position of the sonication point 138.

The medical instrument 100 is further shown as containing an optical unit 150 and a pump unit 152. A catheter 140 is shown as being connected to the optical unit 150 and the pump unit 152. The catheter 140 is a urinary catheter. The catheter 140 has a distal end 144 which is inserted into the bladder 142 of the subject 118. The bladder 142 is adjacent to the target region 139.

In this example the catheter 140 has a balloon 146 that is inflated and keeps the distal end 144 from leaving the bladder 142. The pump unit 152 is able to pump fluid either in or out of the bladder 142. It has two reservoirs, a waste reservoir 154 for receiving fluid that is pumped out of the bladder 142 and a fluorescent dye reservoir 156 for fluid that is pumped into the bladder 142.

In some examples, the catheter 140 only has a main tube. In this case material is both pumped into and out of the bladder 142 by the main tube. In other examples there may be a main tube for removing fluid from the bladder 142 and an auxiliary tube for pumping fluid into the bladder 142. Running along the length of the catheter 140 is also an optical fiber cable 148. It is shown as being connected or coupled to the optical unit 150. The optical unit 150 comprises a light source 158 and a spectrometer 160. Another type of light sensor such as a monochromater may be used in different examples. In some examples there is a separate optical fiber for the light source 158 and the spectrometer 160. In other examples the light source and the spectrometer share a single fiber optic.

The magnetic field gradient coil power supply 112, the high-intensity focused ultrasound system 122, the transceiver 116, the optical unit 150 and the pump unit 152 are shown as being connected to a hardware interface 172 of a computer system 170. The computer system 170 also comprises a processor 174. The processor 174 may actually represent more than one processor and may also represent processors distributed amongst one or more computers. The processor 174 is in communication with the hardware interface 172, a user interface 176, and a memory 178. The hardware interface 172 is an interface which enables the processor 174 to send and receive data and/or commands to the rest of the medical instrument 100 and to control it. The memory 178 may be any combination of volatile or non-volatile memory which the processor 174 has access to.

The memory 178 is shown as containing a set of machine-executable instructions 180 which the processor 174 can use for performing calculations and/or controlling the magnetic resonance imaging system 100.

In this example the high-intensity focused ultrasound system 122 is shown as being integrated with a magnetic resonance imaging system 102. This however is optional. The magnetic resonance imaging system 102 may not be present or may be replaced with another medical imaging system such as a diagnostic ultrasound imaging system or a radiologic imaging system such as a computer tomography imaging system. In this example the high-intensity focused ultrasound system 122 has an adjustable focus 138. The magnetic resonance imaging system is used for guiding the location of the focus 138.

The computer memory 178 is shown as optionally containing a pulse sequence 182. The pulse sequence 182 is a timing diagram or a set of commands which may be used for controlling the magnetic resonance imaging system 102 for acquiring magnetic resonance data. The computer memory 178 is further shown as containing magnetic resonance data 184 that has been acquired by controlling the magnetic resonance imaging system 102 with the pulse sequence 182. The storage 178 is further shown as containing a magnetic resonance image 186 that has been reconstructed from the magnetic resonance data 184. The computer storage 178 is further shown as containing a sonication plan 188 which is a specification of a target region which is to be targeted by the focus 138.

The memory 178 is further shown as containing a fluorescence spectrum 190 that is measured with the spectrometer 160. The computer memory 178 is further shown as containing a bladder temperature 192 that was calculated from the fluorescence spectrum 190. The computer memory 178 is further shown as containing sonication commands 194. The sonication commands are commands used by the processor 174 to control the high-intensity focused ultrasound system to sonicate the target region 139. The sonication commands 194 for instance may be constructed using the bladder temperature 192 as a body core or base temperature and the sonication plan 188. The magnetic resonance image 186 may be optionally used in creating the sonication commands 194 also. The magnetic resonance image 186 may be used for identifying the location of specific anatomic landmarks and may be used for registering the sonication plan 188 to the subject 118.

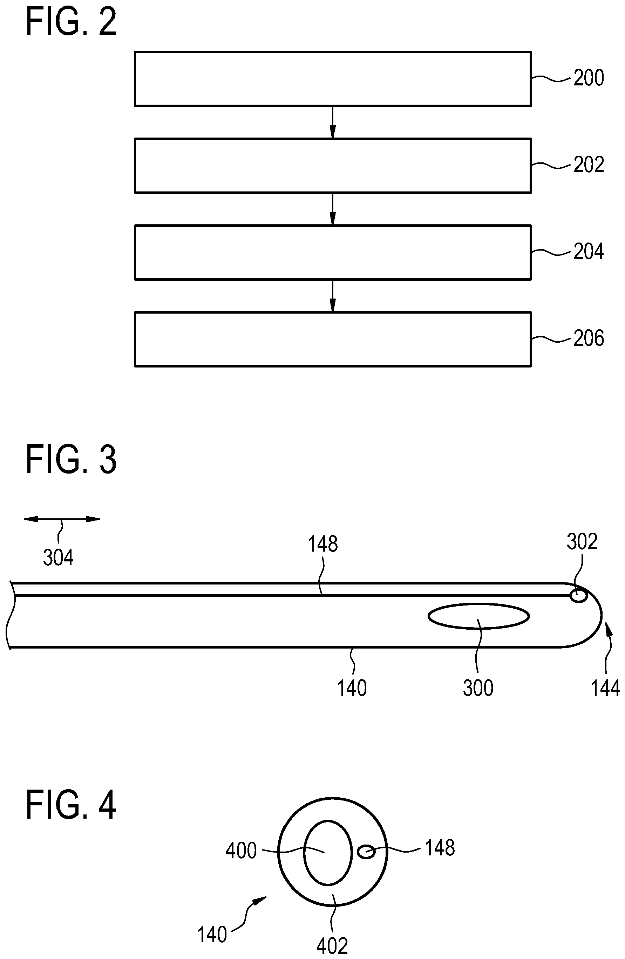

Fig. 2 shows a flowchart of a method which illustrates how to operate the medical instrument 100 of Fig. 1. First in step 200 the sonication plan 188 is received. The sonication plan is descriptive of the sonication of the target region 139. Next in step 204 the fluorescence spectrum 190 is measured using the spectrometer 160. Then in step 204 the bladder temperature 192 is calculated using the fluorescence spectrum 190. Finally in step 206 the sonication commands are generated or calculated by using the sonication plan 188 and the bladder temperature 192.

Fig. 3 shows a side view of a urinary catheter 140. The catheter has an opening 300 near the distal end 144. Also near the distal end 144 can be seen an optical element 302 that is connected to the optical fiber cable 148. In this example there is only a single optical element 302. The light from the light source may be emitted here as well as light being received that is routed to the spectrometer. The catheter 140 has a length direction 304. The fiber optic 148 runs parallel to the length of the catheter 304 for at least a portion of the catheter.

Fig. 4 shows a cross-sectional view of the catheter 140 away from the distal end 144. It can be seen that the catheter has a main tube 400 and a catheter wall 402. The optical fiber cable 148 is in this example embedded within the catheter wall 402.

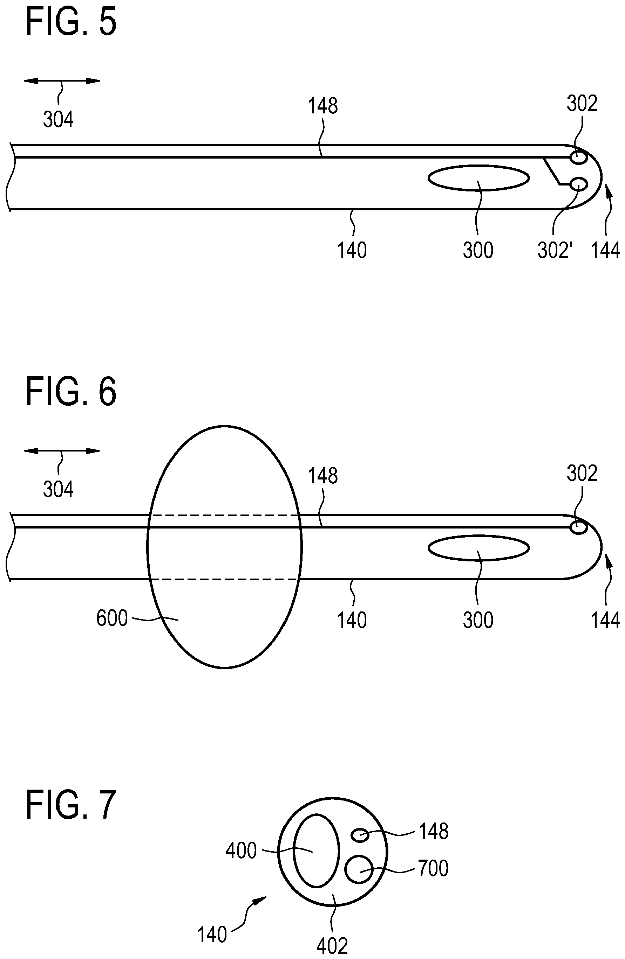

Fig. 5 shows a further example of a catheter 140. The catheter in Fig. 5 is similar to the catheter shown in Fig. 3 except in this case at the distal end 144 there are two optical elements 302 and 302'. One of the optical elements may be for emitting light and the other may be receiving light for the spectrometer. In this case the optical fiber cable 148 may contain two individual fiber optics, one going to optical element 302 and the other going to optical element 302'. The optical elements 302 and 302' may be lenses which may be used to couple the fiber optics more efficiently to the bladder. In the example shown in Fig. 5 the cross-sectional view of the catheter 140 will be equivalent to the cross-sectional view shown in Fig. 4.

Fig. 6 shows a further example of a catheter 140. The catheter 140 is similar to the catheter shown in Fig. 3 except this catheter has a balloon 600 which may be inflated and may be used to prevent the catheter from leaving the bladder. Urinary catheters with such a balloon may be known as a so-called Foley catheter.

Fig. 7 shows a cross-sectional view of a portion of the catheter 140 along the length 304. The catheter can be seen as having two tubes. There is a main tube 400, an inflation tube 700 which is used to provide a saline solution to inflate the balloon 600 and the fiber optic cable 148. All three of these are embedded within the wall 402 of the catheter 140.

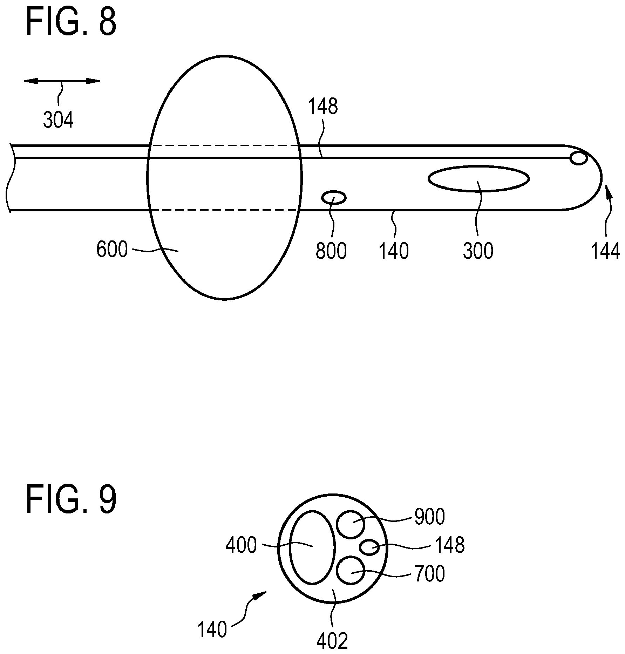

Fig. 8 illustrates a further example of the catheter 140. The catheter shown in Fig. 8 is similar to the catheter shown in Fig. 6 except this catheter also has an outlet 800. At the outlet 800 fluid may be pumped into the bladder. Fluid may then be pumped out or removed through the inlet 300. This for example may be used for efficiently adding the temperature-sensitive fluorescent dye and/or scattering material to fluid within the bladder. It may also be used to efficiently mix the temperature-sensitive fluorescent dye within the bladder. For example fluid could be pumped in at a constant or periodic rate through the outlet 800. Fluid may also be removed at the same rate through the inlet 300. This may serve to efficiently mix fluid within the bladder.

Fig. 9 shows a cross-sectional view of the catheter 140 of Fig. 8. It can be seen that within the wall 402 of the catheter there is a main tube 400 which is connected to the outlet 300. The inflation tube 700 which is connected to the balloon 600 is still present. There is also an auxiliary tube 900 which is connected to the outlet 800. Also shown is the fiber optic cable 148.

The body core temperature can change during the MR-HIFU ablation of uterine fibroid caused by blankets on the patient or air conditioner of the MR scanner. Having a better baseline temperature would lead to a better thermal dose control. Bladder temperature is a good predictor of the core temperature and is near the uterus. By co-injecting Fluorescein and scatter media into bladder long term temperature changes can be detected measuring the frequency shift of the fluorescence in the urine. By fitting the fluorescence spectrum average temperature and temperature variations could be determined without the use of multiple temperature sensors which could interact with the ultrasound field.

As mentioned above, before the start of a MR-HIFU ablation therapy of uterine fibroids the core body temperature is often times determined by ear thermometer. This gives a good estimation of the baseline temperature within and near the uterus at the start of the MR-HIFU therapy. The total treatment time can last for several hours. During the therapy it is not common to perform a second core body temperature measurement. During the procedure the core body temperature can change by applying blankets for comforting the patient, the air conditioner of the MR scanner or physiological changes inside the patient's body. Since the MR is only capable of measuring temperature differences an absolute temperature readout close the target area would improve the thermal dose control.

The core body temperature could be measured by temperature sensors in the rectum, vagina or bladder during the procedure. The the bladder may be better in measuring core body temperature compared to rectal and skin measurements and is nearest to the target area. Since we would like to have baseline temperature we can use the inertia of the urine to measure long term average temperature which is not strongly affected by nearby heating during therapy.

Secondly, when temperature sensors are used their position need to be known avoiding them to be positioned within the beam path of the HIFU transducer. Since the ultrasound absorption coefficient of a liquid is very low it is not expected that the ultrasonic field traveling through the bladder will induce a temperature within the bladder.

Examples may comprise a bladder catheter with optical fibre or optical cable embedded. A lens may be mounted at the end of the fibre to illuminate the full volume of the bladder with, for example, 480nm light. By co-injecting Fluorescein or another temperature dependent fluorescent dye and a scatter medium in the urine, via the catheter, a temperature depended emission spectrum can be observed. The scatter medium can be added to create a more homogenous readout fluorescence through the whole volume of the bladder. Fluorescein is FDA approved and shows a temperature depended: absorption spectrum, relative emitted fluorescence signal and a positive peak shift by increasing temperature when dissolved in water. Especially the peak shift can be used as an absolute temperature readout.

In one example, a standard or modified bladder catheter can be used as well as two optical fibres (excitation and emission) attached within the lumen of the catheter. Via de catheter the Fluorescein as well as scatter media can be injected. A spectrometer can analyze the emission spectrum. Prior injection the emission spectrum can be recorded to remove any background light.

While the invention has been illustrated and described in detail in the drawings and foregoing description, such illustration and description are to be considered illustrative or exemplary and not restrictive; the invention is not limited to the disclosed embodiments.

Other variations to the disclosed embodiments can be understood and effected by those skilled in the art in practicing the claimed invention, from a study of the drawings, the disclosure, and the appended claims. A single processor or other unit may fulfill the functions of several items recited in the claims. The mere fact that certain measures are recited in mutually different dependent claims does not indicate that a combination of these measured cannot be used to advantage. A computer program may be stored/distributed on a suitable medium, such as an optical storage medium or a solid-state medium supplied together with or as part of other hardware, but may also be distributed in other forms, such as via the Internet or other wired or wireless telecommunication systems. Any reference signs in the claims should not be construed as limiting the scope.

### LIST OF REFERENCE NUMERALS

- 100: medical instrument
- 102: magnetic resonance imaging system
- 104: magnet
- 106: bore of magnet
- 108: imaging zone
- 110: magnetic field gradient coils
- 112: magnetic field gradient coils power supply
- 114: radio-frequency coil
- 116: transceiver
- 118: subject
- 120: subject support
- 122: high intensity focused ultrasound system
- 124: fluid filled chamber
- 126: ultrasound transducer
- 128: mechanism
- 130: mechanical actuator/power supply
- 132: path of ultrasound
- 134: ultrasound window
- 136: gel pad
- 138: sonication point
- 139: target region
- 140: catheter
- 142: bladder
- 144: distal end
- 146: balloon
- 148: optical fiber cable
- 150: optical unit
- 152: pump unit
- 154: waste reservoir
- 156: fluorescent dye reservoir
- 158: light source
- 160: light sensor or spectrometer
- 170: computer
- 172: hardware interface
- 174: processor
- 176: user interface
- 178: memory
- 180: machine executable instructions
- 182: pulse sequence
- 184: magnetic resonance data
- 186: magnetic resonance image
- 188: sonication plan
- 190: fluorescence spectrum
- 192: bladder temperature
- 194: sonication commands
- 200: receive a sonication plan descriptive of the sonication of the target region
- 202: measure the fluorescence spectrum using the spectrometer
- 204: calculate a bladder temperature using the fluorescence spectrum
- 206: generate sonication commands using the sonication plan and the bladder temperature
- 300: opening
- 302: optical element
- 304: length of catheter
- 400: main tube
- 402: wall of catheter
- 600: balloon
- 700: inflation tube
- 800: outlet
- 900: auxiliary tube

## Claims

1. A medical instrument (100) comprising:
- a high intensity focused ultrasound system (122) for sonicating a target region (139) within a subject (118);
- a light source (158) for exciting a temperature sensitive fluorescent dye, wherein the light source is configured for coupling to an optical fiber cable (148) of a urinary catheter (140);
- a light sensor (160) for measuring fluorescence (190) emitted by the temperature sensitive fluorescent dye, wherein the light sensor is configured for coupling to the optical fiber cable;
- a fluorescent dye reservoir (156); and
- a pump system (152) configured for connecting to the urinary catheter, wherein the pump system is further connected to the fluorescent dye reservoir, wherein the pump system is configured for pumping from the fluorescent dye reservoir to the distal end of the urinary catheter;
- a memory (178) for storing machine executable instructions (180); and
- a processor (174) for controlling the medical instrument;
wherein execution of the machine executable instructions cause the processor to:
- receive (200) a sonication plan (188) descriptive of a sonication of the target region;
- measure (202) the fluorescence using the light sensor;
- calculate (204) a bladder temperature (192) using the fluorescence; and
- generate (206) sonication commands (194) using the sonication plan and the bladder temperature, wherein the sonication commands are adapted for controlling the high intensity focused ultrasound system to sonicate the target region.

2. The medical instrument of claim 1, wherein the medical instrument comprises the urinary catheter, wherein the optical fiber cable extends from a distal end (144) of the urinary catheter along a length (304) of the urinary catheter, wherein the optical fiber cable is connected to the light source, wherein the optical fiber cable is further connected to the light sensor, and wherein the light source is configured to illuminate the distal end of the urinary catheter using the optical fiber cable.

3. The medical instrument of claim 1 or 2, wherein execution of the machine executable instructions further cause the processor to control the pump system to pump contents of the fluorescent dye reservoir to the distal end of the urinary catheter if the fluorescence has a magnitude below a predetermined threshold.

4. The medical instrument of claim 3, wherein the fluorescent dye reservoir contains the temperature sensitive fluorescent dye and a scattering material.

5. The medical instrument of claim 3 or 4, wherein the scattering material comprises any one of the following: titanium dioxide particles, ultrasound contrast medium, lipide droplets, milk droplets, soya droplets, intralipid droplets, latex particles, and combinations thereof.

6. The medical instrument of any one of the preceding claims, wherein the fluorescent dye is any one of the following: fluroescein, fluorescein I, fluorescein sodium. Indocyanine green, Photofrin, and 5-aminolevulinic acid.

7. The medical instrument of any one of the preceding claims, wherein the sonication commands are further calculated by using the bladder temperature as a starting temperature for the sonication.

8. The medical instrument of claim 7, wherein the sonication plan comprises a specified thermal dose, and wherein the sonication commands are calculated to deliver the specified thermal dose to the target region using the starting temperature.

9. The medical instrument of any one of the preceding claims, wherein execution of the machine executable instructions further causes the processor to control the high intensity focused ultrasound system using the sonication commands.

10. The medical instrument of any one of the preceding claims, wherein execution of the machine executable instructions further cause the processor to:
- repeatedly calculate the bladder temperature during controlling of the high intensity focused ultrasound system, and
- repeatedly correct the sonication commands using the bladder temperature during controlling of the high intensity focused ultrasound system.

11. The medical instrument of any one of the preceding claims, wherein the sonication commands specify multiple sonications, wherein the measurement of the bladder temperature and the generation of the sonication commands is repeated at least once for each of the multiple sonications.

12. The medical instrument of any one of the preceding claims, wherein the high intensity focused ultrasound system has an adjustable focus (138), wherein the medical instrument further comprises a medical imaging system (102), wherein execution of the machine executable instructions further cause the processor to acquire a medical image (186) from an imaging zone, wherein the imaging zone comprises the target region, wherein the sonication commands are adapted for controlling the location of the adjustable focus.

13. A computer program product comprising machine executable instructions (180) for execution by a processor (174) controlling a medical instrument (100), wherein the medical instrument comprises: a high intensity focused ultrasound system (122) for sonicating a target region (139) within a subject (118); a light source (158) for exciting a temperature sensitive fluorescent dye, wherein the light source is configured for coupling to an optical fiber cable (148) of a urinary catheter (140); a light sensor (160) for measuring fluorescence (190) emitted by the temperature sensitive fluorescent dye, wherein the light sensor is configured for coupling to the optical fiber cable; a fluorescent dye reservoir (156); and a pump system (152) configured for connecting to the urinary catheter, wherein the pump system is further connected to the fluorescent dye reservoir, wherein the pump system is configured for pumping from the fluorescent dye reservoir to the distal end of the urinary catheter;
wherein execution of the machine executable instructions cause the processor to:
- receive (200) a sonication plan (188) descriptive of a sonication of the target region;
- measure (202) fluorescence (190) using the light sensor;
- calculate (204) a bladder temperature (192) using the fluorescence; and
- generate (206) sonication commands (194) using the sonication plan and the bladder temperature, wherein the sonication commands are adapted for controlling the high intensity focused ultrasound system to sonicate the target region.

## Patentansprüche

1. Medizinisches Instrument (100), umfassend:
- ein hochintensiv fokussiertes Ultraschallsystem (122) zum Beschallen einer Zielregion (139) innerhalb eines Subjekts (118);
- eine Lichtquelle (158) zum Erregen eines temperaturempfindlichen Fluoreszenzfarbstoffs, wobei die Lichtquelle ausgebildet ist zum Koppeln an ein Lichtwellenleiterkabel (148) eines Harnkatheters (140);
- einen Lichtsensor (160) zum Messen von Fluoreszenz (190), die vom temperaturempfindlichen Fluoreszenzfarbstoff ausgestrahlt wird, wobei der Lichtsensor zum Koppeln an das Lichtwellenleiterkabel ausgebildet ist;
- einen Fluoreszenzfarbstoffbehälter (156); und
- ein Pumpensystem (152), das ausgebildet ist zum Verbinden mit dem Harnkatheter, wobei das Pumpensystem weiter mit dem Fluoreszenzfarbstoffbehälter verbunden ist, wobei das Pumpensystem zum Pumpen vom Fluoreszenzfarbstoffbehälter zum distalen Ende des Harnkatheters ausgebildet ist;
- einen Speicher (178) zum Speichern von maschinenausführbaren Instruktionen (180); und
- einen Prozessor (174) zum Steuern des medizinischen Instruments;
wobei das Abarbeiten der maschinenausführbaren Instruktionen verursacht, dass der Prozessor:
- einen Beschallungsplan (188) empfängt (200), der beschreibend ist für eine Beschallung der Zielregion;
- unter Verwendung des Lichtsensor die Fluoreszenz misst (202);
- unter Verwendung der Fluoreszenz eine Blasentemperatur (192) berechnet (204); und
- unter Verwendung des Beschallungsplans und der Blasentemperatur Beschallungsbefehle (194) erzeugt (206), wobei die Beschallungsbefehle angepasst sind zum Steuern des hochintensiv fokussierten Ultraschallsystems, um die Zielregion zu beschallen.

2. Medizinische Instruments nach Anspruch 1, wobei das medizinische Instruments den Harnkatheter umfasst, wobei sich das Lichtwellenleiterkabel von einem distalen Ende (144) des Harnkatheters entlang einer Länge (304) des Harnkatheters erstreckt, wobei das Lichtwellenleiterkabel mit der Lichtquelle verbunden ist, wobei das Lichtwellenleiterkabel weiter mit dem Lichtsensor verbunden ist, und wobei die Lichtquelle ausgebildet ist, um das distale Ende des Harnkatheters unter Verwendung des Lichtwellenleiterkabels zu beleuchten.

3. Medizinisches Instruments nach Anspruch 1 oder 2, wobei das Abarbeiten der maschinenausführbaren Instruktionen weiter verursacht, dass der Prozessor das Pumpensystem steuert, um Inhalte des Fluoreszenzfarbstoffbehälters an das distale Ende des Harnkatheters zu pumpen, wenn die Fluoreszenz eine Größe unter einem vorbestimmten Schwellenwert aufweist.

4. Medizinisches Instruments nach Anspruch 3, wobei der Fluoreszenzfarbstoffbehälter den temperaturempfindlichen Fluoreszenzfarbstoff und ein Streuungsmaterial enthält.

5. Medizinisches Instruments nach Anspruch 3 oder 4, wobei das Streuungsmaterial eines von Folgendem umfasst: Titandioxidpartikel, UltraschallKontrastmittel, Lipidtröpfchen, Milchtröpfchen, Sojatröpfchen, Intralipidtröpfchen, Latexpartikel und Kombinationen daraus.

6. Medizinisches Instruments nach einem der vorstehenden Ansprüche, wobei der Fluoreszenzfarbstoff eines von Folgendem ist: Fluroeszein, Fluroeszein I, Fluroeszeinnatrium, Indozyaningrün, Photofrin und 5-Aminolävulinsäure.

7. Medizinisches Instruments nach einem der vorstehenden Ansprüche, wobei die Beschallungsbefehle weiter durch Verwenden der Blasentemperatur als Ausgangstemperatur für die Beschallung berechnet werden.

8. Medizinisches Instruments nach Anspruch 7, wobei der Beschallungsplan eine spezifizierte thermische Dosis umfasst, und wobei die Beschallungsbefehle berechnet werden, um die spezifizierte thermische Dosis unter Verwendung der Ausgangstemperatur an die Zielregion abzugeben.

9. Medizinisches Instruments nach einem der vorstehenden Ansprüche, wobei das Abarbeiten der maschinenausführbaren Instruktionen weiter verursacht, dass der Prozessor das hochintensiv fokussierte Ultraschallsystem unter Verwendung der Beschallungsbefehle Anfangstemperatur steuert.

10. Medizinisches Instruments nach einem der vorstehenden Ansprüche, wobei das Abarbeiten der maschinenausführbaren Instruktionen weiter verursacht, dass der Prozessor:
- die Blasentemperatur während des Steuerns des hochintensiv fokussierten Ultraschallsystems wiederholt berechnet, und
- die Beschallungsbefehle unter Verwendung der Blasentemperatur während des Steuerns des hochintensiv fokussierten Ultraschallsystems wiederholt korrigiert.

11. Medizinisches Instruments nach einem der vorstehenden Ansprüche, wobei die Beschallungsbefehle vielfache Beschallungen spezifizieren, wobei die Messung der Blasentemperatur und die Erzeugung der Beschallungsbefehle mindestens einmal für jede der vielfachen Beschallungen wiederholt wird.

12. Medizinisches Instruments nach einem der vorstehenden Ansprüche, wobei das hochintensiv fokussierte Ultraschallsystem eine einstellbare Brennweite (138) aufweist, wobei das medizinische Instrument weiter ein medizinisches bildgebendes System (102) umfasst, wobei das Abarbeiten der maschinenausführbaren Instruktionen weiter verursacht, dass der Prozessor ein medizinisches Bild (186) von einem bildgebenden Bereich erfasst, wobei der bildgebenden Bereich die Zielregion umfasst, wobei die Beschallungsbefehle angepasst sind zum Steuern der Position der einstellbaren Brennweite.

13. Computerprogrammprodukt, umfassend maschinenausführbare Instruktionen (180) zum Abarbeiten durch einen Prozessor (174), der ein medizinisches Instrument (100) steuert, wobei das medizinisches Instruments umfasst: ein hochintensiv fokussiertes Ultraschallsystem (122) zum Beschallen einer Zielregion (139) innerhalb eines Subjekts (118); eine Lichtquelle (158) zum Erregen eines temperaturempfindlichen Fluoreszenzfarbstoffs, wobei die Lichtquelle ausgebildet ist wobei die Lichtquelle ausgebildet ist zum Koppeln an ein Lichtwellenleiterkabel (148) eines Harnkatheters (140); einen Lichtsensor (160) zum Messen von Fluoreszenz (190), die vom temperaturempfindlichen Fluoreszenzfarbstoff ausgestrahlt wird, wobei der Lichtsensor zum Koppeln an das Lichtwellenleiterkabel ausgebildet ist; einen Fluoreszenzfarbstoffbehälter (156); und ein Pumpensystem (152), das ausgebildet ist zum Verbinden mit dem Harnkatheter, wobei das Pumpensystem weiter mit dem Fluoreszenzfarbstoffbehälter verbunden ist, wobei das Pumpensystem zum Pumpen vom Fluoreszenzfarbstoffbehälter zum distalen Ende des Harnkatheters ausgebildet ist;
wobei das Abarbeiten der maschinenausführbaren Instruktionen verursacht, dass der Prozessor:
- einen Beschallungsplan (188) empfängt (200), der beschreibend ist für eine Beschallung der Zielregion;
- unter Verwendung des Lichtsensor die Fluoreszenz (190) misst (202);
- unter Verwendung der Fluoreszenz eine Blasentemperatur (192) berechnet (204); und
- unter Verwendung des Beschallungsplans und der Blasentemperatur Beschallungsbefehle (194) erzeugt (206), wobei die Beschallungsbefehle angepasst sind zum Steuern des hochintensiv fokussierten Ultraschallsystems, um die Zielregion zu beschallen.

## Revendications

1. Instrument médical (100) comprenant :
- un système à ultrasons focalisés de haute intensité (122) pour la sonication d'une région cible (139) à l'intérieur d'un sujet (118) ;
- une source de lumière (158) pour exciter un colorant fluorescent sensible à la température, dans lequel la source de lumière est configurée pour être couplée à un câble à fibre optique (148) d'un cathéter urinaire (140) ;
- un capteur de lumière (160) pour mesurer la fluorescence (190) émise par le colorant fluorescent sensible à la température, dans lequel le capteur de lumière est configuré pour être couplé au câble à fibre optique ;
- un réservoir de colorant fluorescent (156) ; et
- un système de pompe (152) configuré pour être connecté au cathéter urinaire, dans lequel le système de pompe est en outre connecté au réservoir de colorant fluorescent, dans lequel le système de pompe est configuré pour pomper du réservoir de colorant fluorescent vers l'extrémité distale du cathéter urinaire ;
- une mémoire (178) pour stocker des instructions (180) pouvant être exécutées par une machine ; et
- un processeur (174) pour commander l'instrument médical ;
dans lequel l'exécution des instructions pouvant être exécutées par une machine amène le processeur à :
- recevoir (200) un plan de sonication (188) descriptif d'une sonication de la région cible ;
- mesurer (202) la fluorescence à l'aide du capteur de lumière ;
- calculer (204) une température de vessie (192) en utilisant la fluorescence ; et
- générer (206) des commandes de sonication (194) à l'aide du plan de sonication et de la température de vessie, dans lequel les commandes de sonication sont adaptées pour commander le système à ultrasons focalisés de haute intensité pour la sonication de la région cible.

2. Instrument médical selon la revendication 1, dans lequel l'instrument médical comprend le cathéter urinaire, dans lequel le câble de fibre optique s'étend depuis une extrémité distale (144) du cathéter urinaire sur une longueur (304) du cathéter urinaire, dans lequel le câble à fibre optique est connecté à la source de lumière, dans lequel le câble à fibre optique est en outre connecté au capteur de lumière, et dans lequel la source de lumière est configurée pour éclairer l'extrémité distale du cathéter urinaire à l'aide du câble à fibre optique.

3. Instrument médical selon la revendication 1 ou 2, dans lequel l'exécution des instructions pouvant être exécutées par une machine amène en outre le processeur à commander le système de pompe afin de pomper le contenu du réservoir de colorant fluorescent vers l'extrémité distale du cathéter urinaire si la fluorescence a une ampleur inférieure à un seuil prédéterminé.

4. Instrument médical selon la revendication 3, dans lequel le réservoir de colorant fluorescent contient le colorant fluorescent sensible à la température et un matériau diffusant.

5. Instrument médical selon la revendication 3 ou 4, dans lequel le matériau diffusant comprend l'un quelconque des éléments suivants : des particules de dioxyde de titane, un milieu de contraste ultrasonore, des gouttelettes de lipide, des gouttelettes de lait, des gouttelettes de soja, des gouttelettes intralipidiques, des particules de latex et des combinaisons de ceux-ci.

6. Instrument médical selon l'une quelconque des revendications précédentes, dans lequel le colorant fluorescent est l'un des suivants : la fluroescéine, la fluorescéine I, la fluorescéine sodique, le vert d'indocyanine, le Photofrin, et l'acide 5-aminolévulinique.

7. Instrument médical selon l'une quelconque des revendications précédentes, dans lequel les commandes de sonication sont en outre calculées en utilisant la température de vessie comme température de départ pour la sonication.

8. Instrument médical selon la revendication 7, dans lequel le plan de sonication comprend une dose thermique spécifiée, et dans lequel les commandes de sonication sont calculées pour délivrer la dose thermique spécifiée à la région cible en utilisant la température de départ.

9. Instrument médical selon l'une quelconque des revendications précédentes, dans lequel l'exécution des instructions pouvant être exécutées par une machine amène en outre le processeur à commander le système à ultrasons focalisés de haute intensité en utilisant les commandes de sonication.

10. Instrument médical selon l'une quelconque des revendications précédentes, dans lequel l'exécution des instructions pouvant être exécutées par une machine amène en outre le processeur à :
calculer de façon répétée la température de vessie lors de la commande du système à ultrasons focalisés de haute intensité, et
corriger de façon répétée les commandes de sonication en utilisant la température de vessie lors de la commande du système à ultrasons focalisés de haute intensité.

11. Instrument médical selon l'une quelconque des revendications précédentes, dans lequel les commandes de sonication spécifient des sonications multiples, dans lequel la mesure de la température de vessie et la génération des commandes de sonication sont répétées au moins une fois pour chacune des sonications multiples.

12. Instrument médical selon l'une quelconque des revendications précédentes, dans lequel le système à ultrasons focalisés de haute intensité a un foyer réglable (138), dans lequel l'instrument médical comprend en outre un système d'imagerie médicale (102), dans lequel l'exécution des instructions pouvant être exécutées par une machine amène le processeur à acquérir une image médicale (186) à partir d'une zone de formation d'image, dans lequel la zone d'imagerie comprend la région cible, dans lequel les commandes de sonication sont adaptées pour commander l'emplacement du foyer réglable.

13. Programme de programme informatique comprenant des instructions (180) exécutables par une machine, destiné à être exécuté par un processeur (174) commandant un instrument médical (100), dans lequel l'instrument médical comprend : un système à ultrasons focalisés de haute intensité (122) pour la sonication d'une région cible (139) à l'intérieur d'un sujet (118) ; une source de lumière (158) pour exciter un colorant fluorescent sensible à la température, dans lequel la source de lumière est configurée pour être couplée à un câble à fibre optique (148) d'un cathéter urinaire (140) ; un capteur de lumière (160) pour mesurer la fluorescence (190) émise par le colorant fluorescent sensible à la température, dans lequel le capteur de lumière est configuré pour être couplé au câble à fibre optique ; un réservoir de colorant fluorescent (156) ; et un système de pompe (152) configuré pour être connecté au cathéter urinaire, dans lequel le système de pompe est en outre connecté au réservoir de colorant fluorescent, dans lequel le système de pompe est configuré pour pomper du réservoir de colorant fluorescent vers l'extrémité distale du cathéter urinaire ;
dans lequel l'exécution des instructions pouvant être exécutées par une machine amène le processeur à :
- recevoir (200) un plan de sonication (188) descriptif d'une sonication de la région cible ;
- mesurer (202) la fluorescence (190) à l'aide du capteur de lumière ;
- calculer (204) une température de vessie (192) en utilisant la fluorescence ; et
- générer (206) des commandes de sonication (194) à l'aide du plan de sonication et de la température de vessie, dans lequel les commandes de sonication sont adaptées pour commander le système à ultrasons focalisés de haute intensité pour la sonication de la région cible.
